# EUROPÄISCHE PATENTSCHRIFT

(11) **EP 1 075 371 B1**
(45) Veröffentlichungstag und Bekanntmachung des Hinweises auf die Patenterteilung: **28.05.2003**
(21) Anmeldenummer: 99920702.0
(22) Anmeldetag: 19.04.1999
(51) Int. Cl.: B29C 35/08, B29C 35/02, B29C 65/02, B29C 65/78

(54) **VERFAHREN UND VORRICHTUNG ZUM HERSTELLEN VON SEGMENTIERTEN HAFTKLEBESCHICHTEN UND ANBRINGEN AN EINEM SUBSTRAT**
METHOD AND DEVICE FOR PRODUCING SEGMENTED CONTACT ADHESIVE LAYERS AND FOR APPLYING THE SAME ON A SUBSTRATE
PROCEDE ET DISPOSITIF POUR PRODUIRE DES COUCHES AUTO-ADHESIVES SEGMENTEES ET LES APPLIQUER SUR UN SUBSTRAT

(30) Priorität: 07.05.1998 DE 19820366
(43) Veröffentlichungstag der Anmeldung: 14.02.2001
(73) Patentinhaber: Lohmann GmbH & Co. KG, 56567 Neuwied (DE)
(72) Erfinder: ECKER, Günther, D-56567 Neuwied (DE); HOFFEND, Bernd, D-56626 Andemach (DE); HERRMANN, Fritz, D-56567 Neuwied (DE); NITTENWILM, Ralf, D-56203 Höhr-Grenzhausen (DE)
(74) Vertreter: Flaccus, Rolf-Dieter, Dr.
(86) Internationale Anmeldenummer: EP9902612
(87) Internationale Veröffentlichungsnummer: WO99056927

(56) Entgegenhaltungen:
- EP-A- 0 347 741
- EP-A- 0 415 508
- EP-A- 0 748 673
- DE-A- 4 211 448
- DE-U- 29 714 146
- US-A- 3 623 926

## Beschreibung

Die Erfindung betrifft ein Verfahren und eine Vorrichtung zum Herstellen von segmentierten Haftklebeschichten und Anbringen an einem Substrat unter Verwendung eines Reaktionsmediums enthaltend stahleninduzierbare Monomere und/oder Prepolymere olefinisch ungesättigter Verbindungen unter eventueller Zugabe eines Fotoinitiators.

Segmentierte Haftklebeschichten werden in der Praxis vielfach angewendet, beispielsweise bei Haftklebeschichten, für die eine Wasser- oder Gasdurchlässigkeit gefordert wird. Fallweise ist auch die Einsparung von Haftkleber ein Grund für die Segmentierung einer Haftklebeschicht.
Weiterhin ist eine Segmentierung gefordert, wenn kleine Areale auf Substrate verklebt werden. Dabei soll nur das zu verklebende Areal mit einem Segment der Haftklebeschicht ausgerüstet werden.
Als Verfahren zur Segmentierung ist vor allem das Ausstanzen, das Rakeln und der Siebdruck bekannt. Dabei ist das Ausstanzen mit dem Nachteil vergleichsweise großen Abfalls behaftet. Das Rakeln erlaubt nur die Erzeugung begrenzter Schichtdicken, das auch für den Siebdruck zutrifft. Letzterer erfordert darüber hinaus einen beträchtlich Aufwand für die Konturierung.

Das Dokument US 5 344 681 beschreibt ein Verfahren für die Segmentierung, bei dem Haftklebesegmente in Vertiefungen eines Trägermaterials erzeugt werden. Besondere Schwierigkeiten ergeben sich hierbei für das Ausbringen der Segmente aus den Vertiefungen.

Ausgehend vom vorgenannten Stand der Technik liegt der Erfindung die Aufgabe zugrunde, ein Verfahren und eine Vorrichtung anzugeben, die es erlauben, in rationeller Weise segmentierte Haftklebeschichten beliebiger Dicke und Kontur in technisch leicht handhabbarer Weise herzustellen, und die sich für eine Mechanisierung mit hohem Ausbringen unter Verwendung vergleichsweise unkomplizierter Einrichtungen eignen.

Zur Lösung der Aufgabe wird bei einem Verfahren der im Oberbegriff von Anspruch 1 genannten Art mit der Erfindung die nachstehende Folge von Arbeitsschritten vorgeschlagen:
- daß Raktionsmedium unter Sauerstoff-Abschluß in einer Zuteil- und Füllstation bereitgehalten wird,
- daß leere Schablonen mit angelegter unterer Abdeckung ihres segmentbestimmenden Durchbruchs bevorzugt taktweise nacheinander zu einer Füllstation gefördert und darin mit einer dosierbaren Menge des Reaktionsmediums gefüllt werden,
- daß die gefüllten Schablonen in stetigem Transport unter einer UV-Strecke hindurchgeführt und dabei das Reaktionsmedium mittels strahleninduzierter Reaktion unter zumindest teilweiser Polymerisation verfestigt wird,
- daß nach Entfernen der unteren Abdeckung beim weiteren Transport zwischen einer oberen und einer unteren UV-Strecke das dann oben und unten freiliegende Reaktionsmedium von oben und von unten durch fortgesetzte strahleninduzierbare Polymerisationsreaktion zur fertigen Haftklebeschicht durchgehärtet wird,
- daß die die fertige Haftklebeschicht enthaltende Schablone zu einer Übernahmestation transportiert und darin die Haftklebeschicht aus der Schablone herausgedrückt und mit einem Substrat zusammengefügt wird,
- daß gegebenenfalls das ausgerüstete Substrat mit ein Transportband zu einer (nicht gezeigten) Konfektionierungsstation transportiert wird.

Das Verfahren eignet sich, in rationeller Weise und kontinuierlich sowie in vergleichsweise unkomplizierter Art segmentierte Haftklebeschichten beliebiger Dicke und Kontur in technisch leicht handhabbarer Weise herzustellen. Insbesondere eignet sich das Verfahren zur Durchführung auf einer vollmechanisierten vorrichtung.

Eine Ausgestaltung des Verfahrens sieht vor, daß die Dicke der segmentierten Haftklebeschicht durch die Dicke der Schablone festgelegt wird.
Weiterhin ist mit der Erfindung vorgesehen, daß die Schablone und die untere Abdeckung klebstoffabweisend ausgelegt sind, wobei diese aus dehäsivem Material gebildet sind oder die Innenwände des Durchbruchs und die Abdeckung bevorzugt dehäsiv beschichtet werden.
Eine weitere Ausgestaltung des Verfahrens sieht vor, daß die Polymerisationsreaktion durch Bestrahlung des Reaktionsmediums mit UV-Licht oder Elektronenstrahlung durchgeführt und das UV-Licht bevorzugt durch einen Laser erzeugt wird.
Weiterhin ist das Verfahren dadurch gekennzeichnet, daß zur Erzeugung der Haftklebeschicht bevorzugt Haftkleber auf Basis von Acrylaten gewählt werden und das Monomerengemisch vornehmlich unter Zusatz eines Fotoinitiators eingesetzt wird.
Auch kann zur Polymerisation ein Prepolymer eingesetzt werden.
Weil während der Polymerisation das Reaktionsmedium einem Volumenschwund unterworfen wird, sieht eine entsprechende Ausgestaltung des Verfahrens vor, daß dem Reaktionsmedium ein den Volumenschwund im Verlauf der Polymerisation ausgleichender Zusatz beigegeben wird.

Weil das Reaktionsmedium bei seiner Handhabung bis zur Durchführung der Polymerisationsreaktion vor Sauerstoffzutritt geschützt werden muß, hat es sich als vorteilhaft erwiesen, daß die Umgebung der Schablone mit Stickstoff inertisiert wird, um den Umsatz der Reaktion zu erhöhen.
Weil es bei dosierter Zugabe von Reaktionsmedium in eine Durchbrechung der Schablone nicht zwingend erforderlich ist, daß diese vollständig befüllt wird, kann die Dicke des erzeugten Haftklebers nach Maßgabe der zuzumessenden Füllmenge eingestellt werden, das heißt, die Dicke kann kleiner sein als die Dicke der Schablone. Damit ergibt sich für die Fertigung der Klebschicht eine Variationsmöglichkeit in relativ weiten Grenzen, ohne für jede Dicke eine neue Schablone anfertigen zu müssen.

Eine Vorrichtung zum Herstellen von segmentierten Kaftklebeschichten und Anbringen an einem Substrat, insbesondere zum Durchführen des Verfahrens nach der Erfindung ist gekennzeichnet durch die folgenden konstruktiven Merkmale:
- ein endloses Transportband oder -kette für im Kreislauf zu transportierende Schablonen,
- ein innerhalb des Transportbandes unter streckenweiser Ausbildung einer unteren Abdeckung synchron mitlaufendes Unterlagenband,
- eine erste, obere UV-Strecke und eine anschließende untere UV-Strecke,
- eine Übernahmestation mit einer Schablonen-Entleerungs-Einrichtung.

Als Übernahmestation eignet sich ein Drehkreuz.

In der vertikalen Achse ist oberhalb der zu entleerenden Schablone ein Drehkreuz montiert. Von unten wird das konturierte Klebeband mit einem Stempel nach oben aus der Schablone gepreßt. Das Drehkreuz besteht aus 4 im Abstand von 90° angebrachten Auslegern, wobei jeder Ausleger einen Saugfuß und Preßluftzylinder besitzt.
Ein Zyklus beginnt bei 0° am Magazin (4), wo der Sauger das selbstklebend auszurüstende Teil entnimmt. Der Ausleger wird um 90° entgegen dem Uhrzeigersinn gedreht und gelangt an eine Station (5), an der die Oberfläche bearbeitet (z.B. Corona-Vorbehandlung) werden kann. Nach insgesamt 180° wird an der Ausdruck- und Übernahmestation (3) das Teil positionsgenau auf die Schablone gesetzt. Mit Hilfe des Ausdruckstempels an der Unterseite erfolgt nun das gemeinsame Entnehmen des selbstklebend ausgerüsteten Teils. Dieses Teil wird nach einer weiteren Drehung von insgesamt 270° auf einem dehäsiv eingestellten Transportband (6) abgesetzt und anschließend konfektioniert. Nach der letzten 90°-Drehung wird der Zyklus beendet bzw. neu gestartet (360°=0°).

Die Vorrichtung ist vergleichsweise unkompliziert und eignet sich für eine mechanisierte, kontinuierliche Herstellung segmentierter Haftklebeschichten unter Verwendung unkomplizierter technologischer Mittel.

Eine Ausgestaltung der Vorrichtung sieht vor, daß die Schablonen zur Aufnahme von Reaktionsmedium Durchbrechungen aufweisen, die uneingeschränkte segmentbestimmende geometrische Formen aufweisen.
Weiterhin kann eine Ausgestaltung der Vorrichtung dadurch gekennzeichnet sein, daß das Unterlagenband stellenweise segmentförmige Erhebungen aufweist, die in Durchbrechungen von Schablonen hineinragend und deren Befüllung mit Reaktionsmedium verhindernd haftkleberfreie Volumenanteile ausbilden. Diese Erhebungen sind dehäsiv ausgerüstet. Sie bieten die Möglichkeit zur Bildung von allseitig von Haftkleber umgebenen Arealen.
Schließlich sieht die Vorrichtung vor, daß die Schablonen eine Dicke zwischen 0,5 und 6 mm besitzen.

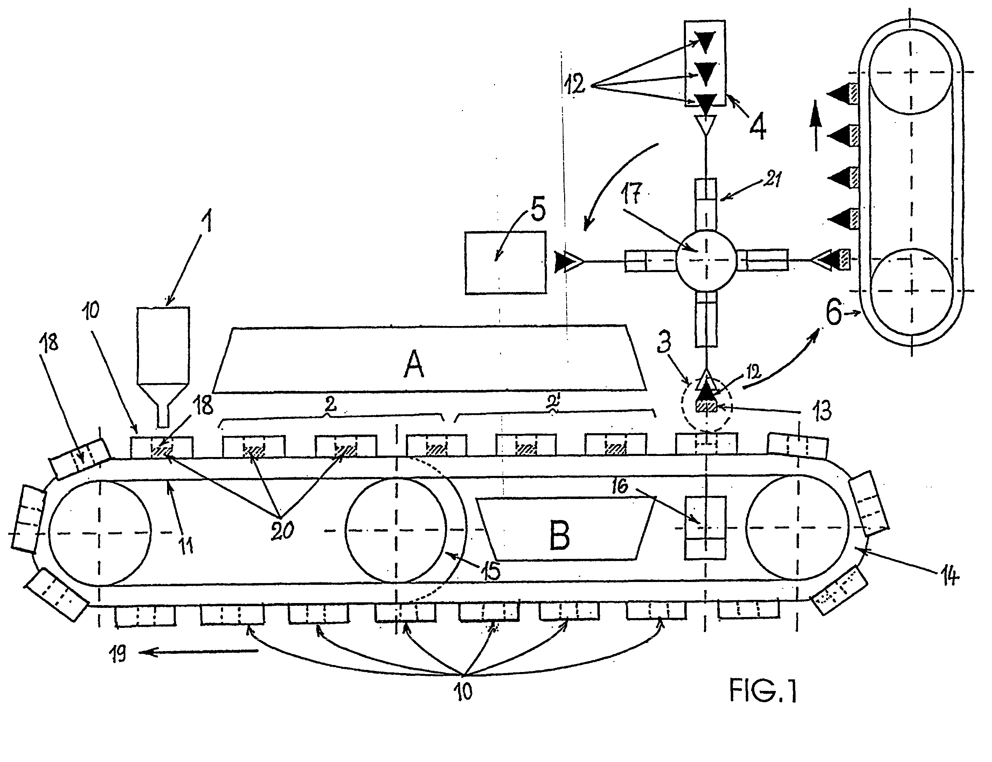

Weitere Einzelheiten, Merkmale und Vorteile der Erfindung ergeben sich aus der nachstehenden Erläuterung eines in den Zeichnungen schematisch dargestellten Ausführungsbeispieles. Die in der Figur dargestellte Vorrichtung umfaßt ein endloses Transportband oder -kette 14 für im Kreislauf zu transportierende Schablonen 10. Diese sind in vorgesehenen gegenseitigen Abständen auf der Transportkette 14 befestigt und laufen gemäß Pfeil 19 im Uhrzeigersinn um jeweils ein Paar Umlenkrollen um. Jede dieser Schablonen 10 weist segmentbestimmende Durchbrechungen 18 auf, die im Bereich der Füllstation 1 durch ein Unterlagenband 15 unter Ausbildung einer unteren Abdeckung 11 an ihrer Unterseite verschlossen wird, so daß das noch flüssige Reaktionsmedium in der Füllstation 1 portionsweise in jede Durchbrechung 18 eingefüllt werden kann. Mit der Bezugsziffer 20 ist die Füllmenge in jeder Schablone 10 gekennzeichnet. Sie kann - wie gezeigt - nur einen Teil der Schablonendicke ausmachen, sie kann aber auch fallweise die Schablone 10 in voller Höhe ausfüllen. Nachdem eine Schablone 10 in der Füllstation 1 mit Reaktionsmedium befüllt wurde, läuft diese in stetigem Transport unter einer UV-Strecke A zunächst bei einem Streckenabschnitt 2 und anschließend daran in Streckenabschnitt 2' zwischen einer oberen UV-Strecke A und einer unteren UV-Strecke B hindurch, wobei das dann oben und unten freiliegende Reaktionsmedium von oben und von unten durch fortgesetzte strahleninduzierbare Polymerisationsreaktion zur fertigen Haftklebeschicht 13 durchgehärtet wird.
Am Schnittpunkt der Transportstrecken 2 und 2' war vorlher das Unterlagenband 15 unter Entfernung der unteren Abdekkung 11 nach unten weggelaufen und hatte somit die Unterseite des Reaktionsmediums zur Polymerisationseinwirkung von unten her freigelegt.
Sodann wird die die fertige Haftklebeschicht 13 enthaltende Schablone 10 zur Übernahmestation 3 transportiert und darin die Haftklebeschicht 13 aus der Schablone 10 mit Hilfe der stempelförmigen Entleerungseinrichtung 16 aus der Schablone 10 herausgedrückt und an die Übernahmestation 3 übergeben. In der Übernahmestation 3 wird mit Hilfe der Schablonen-Entleerungseinrichtung 16 die fertig polymerisierte Haftklebeschicht 13 nach Herausdrücken aus der Schablone 10 an einen Arm des als Drehkreuz ausgebildeten Fördermittels 17 übergeben und beispielsweise durch Saugwirkung darin festgehalten.
Das Drehkreuz 17 entnimmt einem nicht näher dargestellten Vorrat einzelne Substrate 12 und übergibt diese an einen Ausleger, wobei jeder Ausleger einen Saugfuß und Pressluftzylinder besitzt.
Ein Zyklus beginnt bei 0° Drehbewegung am Magazin 4, aus dem der Saugarm 21 das selbstklebend auszurüstende Teil 12 entnimmt. Der Auslegerarm wird sodann um 90° entgegen dem Uhrzeigersinn gedreht und gelangt zur Station 5, an der die Oberfläche eines Teils 12 beispielsweise durch Korona-Vorbehandlung bearbeitet wird. Nach weiterer Drehung um insgesamt 180° wird an der Übernahmestation 3 das Teil 12 positionsgenau auf die Schablone 10 gesetzt, wobei dann mit Hilfe des Ausdruckstempels 16 an der Unterseite das gemeinsame Entnehmen des selbstklebend ausgerüsteten Teils erfolgt.
Dieses Teil 12 zusammen mit der Haftklebeschicht 13 wird nach einer weiteren Drehung des Drehkreuzarmes von insgesamt 270° auf einem dehäsiv eingestellten Transportband 6 abgesetzt und anschließend konfektioniert.
Nach der letzten 90°-Drehung wird der Zyklus beendet und eine neuer Zyklus von 360° gestartet.

Die Erfindung wird anhand der nachfolgenden Beispiele erläutert.

### BEISPIEL 1:

Aus
60 Gewichtsteilen 2-Ethylhexylacrylat
30 Gewichtsteilen Methylacrylat
10 Gewichtsteilen Acrylsäure
0,01 Gewichtsteilen Irgacure 184 (1-Hydroxy-cyclohexylphenylketon)

wird eine ca. 8%ige Polymer-Monomer-Lösung hergestellt. Bei einer Wellenlänge von ca. 280 nm zerfällt der Fotoinitiator, die Reaktion wird gestartet, welche beim Erreichen der Viskosität von ca. 3 Pa.s durch Abschalten der UV-Lampen abgebrochen wird.
Das Prepolymerisat wird anschließend mit 0,6 Gew.% DPGDA (Dipropylenglykoldiacrylat) und 0,6 Gew.% des Fotoinitiators Irgacure 1800 (75 Gew.% 1-Hydroxy- cyclohexylphenylketon und 25 Gew.% Bis(2,6-dimethoxybenzoyl)-2,4,4-trimethylpentylphosphinoxid) versetzt. Durch die Zugabe von 2-Ethylhexylacrylat wird die viskosität geregelt und man erhält das dosierfähige, reaktive Polymer-Monomer-Gemisch, das unter Sauerstoffausschluß gelagert wird.

Dieser Klebstoff wurde in den Hohlraum einer segmentbestimmenden Schablone, in dem ein "L" eingearbeitet ist, dosiert. Der Grundkörper des "L" ist 6 mm breit, der waagerechte und senkrechte Schenkel besitzt eine Länge von 20 bzw. 30 mm. Dieser Hohlraum wurde in eine 1,5 mm dicke Teflonplatte eingefräst.
Die Polymerlösung wird mittels einer Kolbenpumpe und eines Aluminiumrohres (Innendurchmesser 2 mm) in den Hohlraum zwischen silikonisierter Polyesterfolie und der darauf sitzenden Durchbrechung ("L-Form") dosiert. Die Form wird vollständig gefüllt, das Volumen beträgt 0,35 cm³. Die gefüllte Schablone wird in einem mit Stickstoff inertisierten Raum 15 Sekunden lang mit einem UV-Laser bestrahlt.
Typ: Lextra 200 (Fa. Lambda Physik, Göttingen) Wellenlänge: 351 nm, Impulsrate 5-30 Hz, mittlere Leistung 10 W, Aufspreizung 30 mm x 40 mm,
wobei der segmentierte Klebstoff polymerisiert und weitgehend aushärtet. Anschließend dreht man die Schablone um 180° und bestrahlt nochmals 15 Sekunden von der gegenüberliegenden Seite, wobei der Polyacrylatgrundkörper vollständig aushärtet (Umsatz > 99 %). Mit Hilfe eines Negativstempels (L-Form mit einem Schlupf von 0,05 mm) wird das segmentierte transparente Klebeband von der Schablone befreit und auf einer silionisierten PETP-Folie abgesetzt. Ein aus Hart-PVC gefertigtes L-Stück mit gleichen Ausmaßen wird coronavorbehandelt und mit der segmentierten Haftklebstoffschicht verklebt, so daß ein selbstklebendes 1,5 mm dickes Element entsteht.

### BEISPIEL 2:

Radikalische Substanzpolymerisation wie in Beispiel 1.
Aus
90 Gewichtsteilen 2-Ethylhexylacrylat
10 Gewichtsteilen Acrylsäure
0,02 Gewichtsteilen Irgacure 500

wird eine ca. 8%ige Polymer-Monomer-Lösung hergestellt. Eine Zielviskosität von 1,5 Pa.s wird eingestellt. Bei einer Wellenlänge von ca. 200-280 nm zerfällt der Fotoinitiator, die Reaktion wird gestartet, welche beim Erreichen der Viskosität von ca. 1,5 Pa.s durch Abschalten der UV-Lampen abgebrochen wird.

Das Prepolymerisat wird anschließend mit 0,6 % HDDA (Hexandioldiacrylat) und 0,8 % des Fotoinitiators Irgacure 1700 (75 Gew.% 1-Hydroxy-2-methyl-1-phenyl-propan-1-on und 25 Gew.% Bis (2,6-dimethoxybenzoyl)-2,4,4-trimethylpentylphosphinoxid) versetzt. Durch die Zugabe von 1,5 Gew.% Mikrohohlglaskugeln Q-CEL ® 300 (US 4,223,067) erhält man ein gefülltes dosierfähiges und reaktives Polymer-Monomer-Gemisch, das unter Sauerstoffausschluß gelagert wird.
Es wird die L-Schablone von Beispiel 1 verwendet. Die Form wird nur teilweise gefüllt, das Volumen beträgt 0,24 cm³. Die Polymerlösung wird mittels einer Kolbenpumpe und eines Aluminiumdruckrohres (Innendurchmesser 2 mm) in den Hohlraum zwischen silikonisierter Polyesterfolie und der darauf sitzenden Durchbrechung ("L-Porm") dosiert. Die Form wird vollständig gefüllt, das Volumen beträgt 0,35 cm³. Die gefüllte Schablone wird in einem mit Stickstoff inertisierten Raum 30 Sekunden lang mit einer UV-Lampe bestrahlt.
Typ: UVA-Leuchtstofflampe TL-K40W/10R mit Reflektor, Leistung 40 W, Wellenlänge 315-400 nm, Philips,
wobei der segmentierte Klebstoff polymerisiert und weitgehend aushärtet. Anschließend dreht man die Schablone um 180° und bestrahlt nochmals 15 Sekunden von der gegenüberliegenden Seite, wobei der Polyacrylatgrundkörper vollständig aushärtet (Umsatz > 99 %). Mit Hilfe eines Negativstempels (L-Form mit einem Schlupf von 0,05 mm) wird das segmentierte, weiße Klebeband von der Schablone befreit und auf einer silikonisierten PETP-Folie abgesetzt. Ein aus Hart-PVC gefertigtes L-Stück mit gleichen Ausmaßen wird coronavorbehandelt und mit der segmentierten Haftklebstoffschicht verklebt, so daß ein 1,0 mm dickes selbstklebendes Element entsteht.

Durch die Erhöhung der Dichte beim Übergang vom Monomer zum Polymer kommt es zu einer Volumenkontraktion und damit zu einer leichten Krümmung der oberen Fläche der segmentierten Haftklebeschicht. Dieses Phänomen kann man mit geringen Dosen von gängigen Expandierungsmitteln wie Backpulver oder Expancel 551DU der Fa. Nobel Industries, Schweden, ausgleichen.

Das erfindungsgemäße Verfahren und die zu seiner Durchführung vorgesehene Vorrichtung sind unkompliziert, zweckmäßig und wirtschaftlich durchführbar. Insofern löst die Erfindung in optimaler Weise die eingangs gestellte Aufgabe.

## Patentansprüche

1. Verfahren zum Herstellen von segmentierten Haftklebeschichten und Anbringen an einem Substrat unter Verwedung eines Reaktionsmediums enthaltend strahleninduzierbares Polymerisat und/oder Prepolymere olefinisch ungesättigter Verbindungen unter Zugabe eines Fotoinitiators, **gekennzeichnet durch** die Arbeitsschritte:
- daß Reaktionsmedium unter Sauerstoff-Abschluß in einer Zuteil- und Füllstation (1) bereitgehalten wird,
- daß leere Schablonen (10) mit angelegter unterer Abdekkung (11) ihres segmentbestimmenden Durchbruchs (18) bevorzugt taktweise nacheinander zu einer Füllstation (1) gefördert und darin mit einer dosierbaren Menge des Reaktionsmediums gefüllt werden,
- daß die gefüllten Schablonen (10) in stetigem Transport unter einer UV-Strecke (A) hindurchgeführt und dabei das Reaktionsmedium mittels strahleninduzierter Reaktion unter zumindest teilweiser Polymerisation verfestigt wird,
- daß nach Entfernen der unteren Abdeckung (11) beim weiteren Transport zwischen einer oberen (A) und einer unteren UV-Strecke (B) das dann oben und unten freiliegende Reaktionsmedium von oben und von unten **durch** fortgesetzte strahleninduzierbare Polymerisationsreaktion zur fertigen Haftklebeschicht (13) durchgehärtet wird,
- daß die die fertige Haftklebeschicht (13) enthaltende Schablone (19) zu einer übernahmestation (3) transportiert und darin die Haftklebeschicht (13) aus der Schablone (10) herausgedrückt und mit einem Substrat (12) zusammengefügt wird.

2. Verfahren nach Anspruch 1, **dadurch gekennzeichnet, daß** das mit der Haftklebschicht (13) zusammengefügte Substrat (12) an ein Drehkreuz (17) übergeben und gegebenenfalls das ausgerüstete Substrat (12) mit einem Transportband (6) zu einer Konfektionierungsstation transportiert wird.

3. Verfahren nach Anspruch 1 oder 2, **dadurch gekennzeichnet, daß** die Dicke der segmentierten Haftklebeschicht durch die Dicke der Schablone (10) festgelegt wird.

4. Verfahren nach einem oder mehreren der Ansprüche 1 bis 3, **dadurch gekennzeichnet, daß** die Schablone (10) und die untere Abdeckung (11) klebstoffabweisend ausgerüstet werden.

5. Verfahren nach einem oder mehreren der Ansprüche 1 bis 4, **dadurch gekennzeichnet, daß** die Innenwände des Durchbruchs (13) und die Abdeckung (11) dehäsiv beschichtet werden.

6. Verfahren nach einem oder mehreren der Ansprüche 1 bis 5, **dadurch gekennzeichnet, daß** die Polymerisationsreaktion durch Bestrahlung des Reaktionsmediums mit UV-Licht oder Elektronenstrahlung durchgeführt und das UV-Licht bevorzugt durch einen Laser erzeugt wird.

7. Verfahren nach einem oder mehreren der Ansprüche 1 bis 6, **dadurch gekennzeichnet, daß** zur Erzeugung der Eaftklebeschicht bevorzugt Haftkleber auf Basis von Acrylaten gewählt werden und das Monomerengemisch gegebenenfalls unter Zusatz eines Fotoinitiators eingesetzt wird.

8. Verfahren nach einem oder mehreren der Ansprüche 1 bis 7, **dadurch gekennzeichnet, daß** zur Polymerisation ein Prepolymer eingesetzt wird.

9. Verfahren nach einem oder mehreren der Ansprüche 1 bis 8, **dadurch gekennzeichnet, daß** dem Reaktionsmedium ein den Volumenschwund im Verlauf der Polymerisation ausgleichender Zusatz beigegeben wird.

10. Vorrichtung zum Herstellen von segmentierten Haftklebeschichten und Anbringen an einem Substrat, insbesondere zur Durchführung des Verfahrens nach der Erfindung, **gekennzeichnet durch** die konstruktiven Merkmale:
- ein endloses Transportband oder -kette (14) für im Kreislauf zu transportierende Schablonen (10),
- ein innerhalb des Transportbandes (14) unter streckenweiser Ausbildung einer unteren Abdeckung (11) synchron mitlaufendes Unterlagenband (15),
- eine erste, obere UV-Strecke (A) und eine anschließende untere UV-Strecke (B),
- eine Übernahmestation (3) mit einer Schablonen-Entleerungseinrichtung (16).

11. Vorrichtung nach Anspruch 10
- mit einem als Drehkreuz ausgebildeten Fördermittel (17),
- einer Aufgabestation (4) für Substrate (12) an das Dreh kreuz (17),
- einer Bearbeitungsstation (5) im Bereich des Drehkreuzes (17),
- einem mit dem Drehkreuz (17) zusammenwirkenden dehäsiv ausgerüsteten Transportband (6).

12. Vorrichtung nach Anspruch 10 oder 11, **dadurch gekennzeichnet, daß** die Schablonen (10) zur Aufnahme von Reaktionsmedium Durchbrechungen (18) aufweisen, die uneingeschränkte segmentbestimmende geometrische Formen aufweisen.

13. Vorrichtung nach einem oder mehreren der Ansprüche 10 bis 12, **dadurch gekennzeichnet, daß** das Unterlagenband (15) stellenweise segmentförmige Erhebungen aufweist, die in Durchbrechungen (18) von Schablonen (10) hineinragend und deren Befüllung mit Reaktionsmedium verhindernd haftkleberfreie Volumenanteile ausbilden.

14. Vorrichtung nach einem oder mehreren der Ansprüche 10 bis 13, **dadurch gekennzeichnet, daß** die Erhebungen (19) dehäsiv ausgerüstet sind.

15. Vorrichtung nach einem oder mehreren der Ansprüche 10 bis 14, **dadurch gekennzeichnet, daß** die Schablonen (10) eine Dicke zwischen 0,5 und 6 mm besitzen.

## Claims

1. Process for manufacturing segmented pressure-sensitive adhesive layers and for applying the same to a substrate, using a reaction medium containing radiation-induced polymers and/or prepolymers of olefinically unsaturated compounds under addition of photoinitiators, **characterized by** the following steps:
- that the reaction medium is kept ready in a metering and filling station (1) under absence of oxygen,
- that the empty templates (10) are conveyed, preferably intermittently one after another, with a lower covering (11) of their segment-defining opening (18) put against the same, to a filling station (1) wherein they are filled with a dosable amount of the reaction medium,
- that the filled templates (10) are passed in continuous conveyance below a UV section (A), whereby the reaction medium is solidified by means of radiation-induced reaction involving at least partial polymerisation,
- that after removal of the lower covering (11) the reaction medium, which is then exposed on the upper side and the bottom side, is, during the further transport between an upper (A) and a lower UV section (B), fully cured from above and from below by continued radiation-induced polymerisation reaction to form the finished pressure-sensitive adhesive layer (13),
- that the template (19), containing the finished pressure-sensitive adhesive layer (13), is conveyed to a transfer station (3), wherein the pressure-sensitive adhesive layer (13) is pushed out of the template (10) and is combined with a substrate (12).

2. Process according to Claim 1, **characterized in that** the substrate (12) which has been combined with the pressure-sensitive adhesive layer (13) is delivered to a turnstile (17), and that the substrate (12), which is equipped with the pressure-sensitive adhesive layer (13), is optionally conveyed by a conveyor belt (6) to a final-manufacturing station.

3. Process according to Clam 1 or 2, **characterized in that** the thickness of the segmented pressure-sensitive adhesive layer is defined by the thickness of the templates (10).

4. Process according to one or more of Claims 1 to 3, **characterized in that** the template (10) and the covering (11) are rendered anti-adhesive.

5. Process according to one or more of claims 1 to 4, **characterized in that** the inner walls of the opening (13) and the covering (11) are provided with a dehesive coating.

6. Process according to one or more of claims 1 to 5, **characterized in that** the polymerisation reaction is performed by irradiating the reaction medium with ultraviolet light or electron radiation and that the ultraviolet light is preferably generated by a laser.

7. Process according to one or more of claims 1 to 6, **characterized in that** to manufacture the pressure-sensitive adhesive layer, acrylate-based pressure-sensitive adhesives are selected with preference, and that the mixture of monomers is optionally used under addition of a photoinitiator.

8. Process according to one or more of claims 1 to 7, **characterized in that** a prepolymer is used for polymerisation.

9. Process according to one or more of claims 1 to 8, **characterized in that** an additive is added to the reaction medium, which additive compensates the volume shrinkage in the course of the polymerisation.

10. Device for manufacturing segmented pressure-sensitive adhesive layers and applying the same to a substrate, and, in particular, for performing the process according to the invention, **characterized by** the constructional features:
- a continuous conveyor belt or chain (14) for templates (10) to be conveyed in circulation,
- a support or base belt (15) running synchronously within the said conveyor belt (14) under section-wise formation of a lower covering (11),
- a first, upper UV section (A) and a subsequent lower UV section (B),
- a transfer station (3) comprising a template-emptying device (16).

11. Device according to Claim 10, comprising
- conveyor means (17) configured as a turnstile
- a feed station (4) for feeding substrates (12) to the turnstile (17),
- a processing station (5) in the region of the turnstile (17),
- a conveyor belt (6), which has been rendered dehesive, cooperating with the turnstile (17),

12. Device according to Claim 10 or 11, **characterized in that** the templates (10) have openings (18) to receive the reaction medium, said opeaings (18) having unlimited, segment-defining geometric shapes.

13. Device according to one or more of Claims 10 to 12, **characterized in that** the support or base belt (15) in places has segment-shaped protuberances which form pressure sensitive adhesive-free volume portions by projecting into the openings (18) of templates (10) and preventing that the same are filled with reaction medium.

14. Device according to one or more of Claims 10 to 13, **characterized in that** the protuberances (19) are rendered dehesive.

15. Device according to one or more of Claims 10 to 14, **characterized in that** the templates (10) have a thickness of between 0.5 to 6 mm.

## Revendications

1. Procédé pour produire des couches auto-adhésives segmentées et leur application sur un substrat en utilisant un agent réactif contenant des polymères et/ou des prépolymères inductibles par rayons de composés non saturés en oléfines en ajoutant un photoinitiateur, **caractérisé par** les étapes suivantes
- tenir disponible l'agent réactif sous exclusion d'oxygène dans une station de dosage et de remplissage (1),
- transporter des moules (10) vides successivement, de préférence de manière cadencée, avec couverture inférieure (11) de leur creux (18) déterminant les segments vers une station de remplissage (1) et les remplir dans cette station avec une quantité dosable de l'agent réactif,
- transporter continuellement les moules (10) remplis sous un tronçon UV (A) pour ainsi solidifier l'agent réactif à l'aide d'une réaction induite par rayons avec une polymérisation du moins partielle,
- après enlèvement de la couverture inférieure (11) lors du transport entre un tronçon UV supérieur (A) et infërieur (B), durcir d'en haut et d'en bas l'agent réactif alors découvert en haut et en bas à l'aide dune réaction de polymérisation continue pouvant étre induite par rayons, pour obtenir la couche auto-adhésive (13) finie,
- transporter le moule (10) contenant la couche auto-adhésive (13) finie vers une station de transfert (3), pour y extraire par pression la couche auto-adhésive (13) du moule (10) et l'assembler avec un substrat (12).

2. Procédé selon la revendication 1, **caractérisé en ce que** le substrat (12) assemblé avec la couche auto-adhésive (13) est transféré à un tourniquet (17), et **en ce que** le cas échéant le substrat (12) équipé est transporté à l'aide d'une bande de transport (6) vers une station de confection.

3. Procédé selon la revendication 1 ou 2, **caractérisé en ce que** l'épaisseur de la couche auto-adhésive segmentée est déterminée par l'épaisseur du moule (10).

4. Procédé selon l'une ou plusieurs des revendications 1 à 3, **caractérisé en ce que** le moule (10) et la couverture inférieure (11) sont conçus anti-adhésifs

5. Procédé selon l'une ou plusieurs des revendications 1 à 4, **caractérisé en ce que** les parois inférieures du creux (13) et la couverture (11) présentent un revêtement anti-adhésif.

6. Procédé selon l'une ou plusieurs des revendications 1 à 5, **caractérisé en ce que** la réaction de polymérisation est réalisée par exposition de l'agent réactif au rayonnement de lumière UV ou sous rayonnement d'électrodes, et **en ce que** la lumière UV est de préférence générée par un laser.

7. Procédé selon l'une ou plusieurs des revendications 1 à 6, **caractérisé en ce que** pour obtenir la couche auto-adhésive, on choisit de préférence une colle adhésive à base d'acrylates, et **en ce que** le cas échéant le mélange de monomères est mis en oeuvre en ajoutant un photoinitiateur.

8. Procédé selon l'une ou plusieurs des revendications 1 à 7, **caractérisé en ce que** pour la polymérisation on utilise un prépolymère.

9. Procédé selon l'une ou plusieurs des revendications 1 à 8, **caractérisé en ce qu'**un additif compensant la perte de volume en cours de polymérisation est ajouté à l'agent réactif.

10. Dispositif pour produire des couches auto-adhésives segmentées et les appliquer sur un substrat, notamment pour la mise en oeuvre du procédé selon l'invention, **caractérisé par** les caractéristiques constructives suivantes:
- une bande ou une chaîne de transport sans fin (14) pour des moules (10) transportés dans un mouvement circulaire,
- une bande de support (15) à mouvement conjoint synchronisé à l'intérieur de la bande de transport (14) en formant par tronçons une couverture inférieure (11),
- un premier tronçon UV supérieur (A) suivi d'un tronçon UV inférieur (B),
- une station de transfert (3) comprenant un dispositif d'extraction de moule (16)

11. Dispositif selon la revendication 10, comprenant :
- un moyen de transport (17) sous la forme d'un tourniquet,
- une station d'envoi (4) de substrats (12) vers le tourniquet (17),
- une station de traitement (5) dans la zone du tourniquet (17),
- une bande de transport (6) anti-adhésive coopérant avec le tourniquet (17).

12. Dispositif selon la revendication 10 ou 11, **caractérisé en ce que** les moules (10) présentent des creux (18) pour recevoir un agent réactif, qui présentent des formes géométriques quelconques déterminant les segments.

13. Dispositif selon l'une ou plusieurs des revendications 10 à 12, **caractérisé en ce que** la bande de support (15) présente à certains endroits des élévations en forme de segments qui, saillant dans des creux (18) de moules (10) et empêchant le remplissage de ceux-ci par un agent réactif, forment des parts de volume sans colle adhésive.

14. Dispositif selon l'une ou plusieurs des revendications 10 à 13, **caractérisé en ce que** les élévations (19) sont anti-adhésives.

15. Dispositif selon l'une ou plusieurs des revendications 10 à 14, **caractérisé en ce que** les moules (10) présentent une épaisseur comprise entre 0,5 et 6 mm.
